## Europäisches Patentamt
## European Patent Office
## Office européen des brevets

(19)

(11) Veröffentlichungsnummer: **0 294 516**
**B1**

(12)

# EUROPÄISCHE PATENTSCHRIFT

(45) Veröffentlichungstag der Patentschrift:
**25.10.89**

(51) Int. Cl.⁴: **A61G 9/00**

(21) Anmeldenummer: **87118101.2**

(22) Anmeldetag: **08.12.87**

(54) **Vorrichtung zur Entleerung und Reinigung von Hygienegefässen aus einer Spülkammer mit konischem Unterteil, mit Geruchsabschluss, sowie mit Reinigungsdüsen in der Spülkammer und mit einer horizontalen oder vertikalen Tür.**

(43) Veröffentlichungstag der Anmeldung:
**14.12.88 Patentblatt 88/50**

(45) Bekanntmachung des Hinweises auf die Patenterteilung:
**25.10.89 Patentblatt 89/43**

(84) Benannte Vertragsstaaten:
**AT CH DE GB IT LI NL**

(56) Entgegenhaltungen:
EP-A- 0 093 846
DE-A- 3 232 329
DE-C- 500 327
FR-A- 2 416 007
GB-A- 1 168 545
GB-A- 1 181 133

(73) Patentinhaber: **SIC AG, Wartenbergstrasse 15 Postfach, CH-4020 Basel(CH)**

(72) Erfinder: **Harlegard, Jan, Dipl.-Ing., Fichtenrain 5, CH-4106 Therwil/ bei Basel(CH)**

(74) Vertreter: **Jahn-Held, Wilhelm W. Dr.Dr.-Ing. Dipl.-Chem., Schöne Aussicht 8, D-3513 Staufenberg-Landwehrhagen(DE)**

ACTORUM AG

## Beschreibung

Die Erfindung hat sich die Aufgabe gestellt, eine Vorrichtung zur Entleerung und Reinigung und gegebenenfalls zur Desinfektion von Hygienegefässen im Pflegesektor ohne Kontaminierung der äusseren Vorrichtung zu entwickeln.

Diese Aufgabe hat für die Hygiene und Sicherung gegen Infektionen im Krankenhaus besondere Bedeutung.

Aus der schweizer Patentschrift 602 202 ist eine Vorrichtung zum Spülen und Desinfizieren von Steckbecken bekannt, die ein Gestell mit einer Spülkammer beschreibt, die unten mit einem Ablauf verbunden ist und oben eine Öffnung aufweist, die mit einem horizontal verschiebbearem Deckel verschliessbar ist. In der Spülkammer ist ein Ringkanal mit einem Auslasspalt vorhanden. Unter dem Ringkanal ist eine Anzahl Sprühdüsen verteilt. Der Ringkanal und ie Sprühdüsen sind mit der Kalt- und Heisswasser-Druckleitung verbunden. Der Innenraum der Spülkammer ist mit einer ausserhalb von dieser befindlichen Vorrichtung zur Dampfkondensation verbunden.

Der zu reinigen Behälter muss durch eine Pflegeperson in die Spülkammer eingebracht werden. Dann bestätigt diese Person einen Schalter, der die Programmsteuerung in Gang setzt zur Steuerung des Arbeitsablaufes.

Diese bekannte Vorrichtung hat verschiedene Nachteile. Wenn ein Kot enthaltendes Steckbecken gereinigt werden soll, muss die Pflegeperson das Steckbecken zunächst mit der Hand auskippen und in die Spülkammer entleeren. Dabei ist nicht zur verhindern, dass eine äusserliche Beschmutzung und eine bakterielle Infektion eintreten kann. Diese Vorricvhtung hat auch den technischen Nachteil eines grossen Platzbedarfes, da beim Öffnen der Deckel in horizontaler Richtung verschoben werden muss.

Diese Nachteile werden durch die Vorrichtung nach dem europäischen Patent 0 093 846 vermieden. Dieses beschreibt eine Vorrichtung aus einer Spülkammer, die auf einer Seite eine, durch eine Schiebetür verschliessbare Öffnung und eine zum Halten eines Hygienegefässes dienende, um eine horizontale Schwenkachse schwenkbare Drehvorrichtung aufweist. Die Stufen zur Entleerung, Reinigung und Desinfektion werden programmgesteuert. Mit dieser bekannten Vorrichtung wird nach der Entleerung des Gefässes mittels der Drehvorrichtung über eine Drehdüse an der Decke der Spülkammer der Innenraum der Spülkammer und die Aussenflächen des in die Drehvorrichtung eingesetzen HYgienegefässes gereinigt und danach über eine Drehkupplung die mit der Drehvorrichtung fest und auswechselbar verbunden ist, Druckwasser über eine Düse, die sich über der Mitte des Hygienegefässes befindet, intensiv und optimal eingespritzt, und das Reinigungswasser im unteren, konischen Teil der Spülkammer entfernt. Es erfolgt danach als angeschlossene Arbeitsstufe die Desinfektion der entleerten und gereinigten Hygienegefässe mittels Dampf, woran sich die Öffnung der Tür anschliesst unter gleichzeitiger Schwenkung der Befestigungshalterung in der Drehvorrichtung in umgekehrter Schwenkrichtung zur Entnahme des leeren, gereinigten und desinfizierten Hygienegefässes.

Diese Vorrichtung hat sich in der Praxis bewährt. Es ist nun eine weitere Aufgabe der Erfindung, diese Vorrichtung durch eine konstruktiv einfachere Ausführung zu ergänzen, die keine solche Drehvorrichtung mit Befestigungshalterung in der Spülkammer benötigt.

Weiter ist es eine Aufgabe der Erfindung, die Durchsatzkapazität durch den Einsatz von 2 Urinoder Sekret-Flaschen zu erhöhen.

Gegenstand der Vorrichtung der Erfindung ist eine Vorrichtung zur Entleerung und Reinigung von Hygienegefässen aus einer Spülkammer mit konischem Unterteil, mit Geruchabschluss, sowie mit Reinigungsdüsen in der Spülkammer und mit einer horizontalen oder vertikalen Tür.

Die Vorrichtung der Erfindung ist im Oberbegriff des Anspruches 1 definiert.

Die Lösung der Aufgabe der Erfindung ist im kennzeichnenden Teil des Anspruches 1 definiert.

Die Vorrichtung der Erfindung ist dadurch gekennzeichnet

- daß diese Tür eine Klapptür (5) ist und eine Drehachse (6) an ihrem unteren Rand aufweist, wobei die Klapptür in ihrem inneren Hohlraum ein Rohrleitungssystem (7) mit zwei oder mehreren, auf der Innenseite der Tür angeordneten Düsen (9, 9a) und mit einem Verbindungsstück (10) aufweist, und die Klapptür in der Drehachse nach oben zugeklappt, oder motorisch geschlossen wird,

- daß das Verbindungsstück in einen Verbindungsstutzen (11) einführbar ist, der über eine im Spülraum oder nach aussen geführte Rohrleitung (12) an das Rohrleitungssystem (7) der Spülkammer (1) angeschlossen ist,

- daß nach dem Einschalten des Reinigungswassers, der Wasserstrahl durch die Düsen vollständig auf die Unterseite des Spülgutes, wie Pflegegefäße (8) gerichtet ist, und gegebenenfalls beim Einsetzen eines Deckels eine (9a) der Düsen den Wasserstrahl auch auf die Unterseite des Deckels richtet,

- daß die allseitige Reinigung des Spülgutes durch die Reinigungsdüse (7) über die Verbindungsleitung (16) zum Reinigungssystem erfolgt, oder durch die weiteren Düsen (4, 4a) in der Spülkammer unterstützt wird,

- daß gereinigte und gegebenenfalls desinfizierte Gefäße als Spülgut entnommen werden können, wenn nach Beendigung des Reinigungsvorganges und Abstellen der Wasserzufuhr, die Klapptür (5) geöffnet wird, und

- daß der Reinigungsvorgang in gleicher Weise wiederholbar ist.

Die Vorrichtung der Erfindung ist auch dadurch gekennzeichnet,

- daß diese Tür eine Klapptür (5) ist und eine Drehachse (6) an ihrem unteren Rand aufweist, wobei die Klapptür in ihrem inneren Hohlraum ein Rohrleitungssystem (7) mit zwei oder mehreren, auf der Innenseite der Tür angeordneten Düsen (9, 9a) und mit einem Verbindungsstück (10) aufweist, und die Klapptür in der Drehachse nach oben zugeklappt, oder motorisch geschlossen wird,

– daß das Verbindungsstück ein flexibler Schlauch ist, der über eine im Spülraum oder nach aussen geführte Rohrleitung (12) an das Rohrleitungssystem (7) der Spülkammer (1) angeschlossen ist,

– daß nach dem Einschalten des Reinigungswassers, der Wasserstrahl durch die Düsen vollständig auf die Unterseite des Spülgutes, wie Pflegegefäße (8) gerichtet ist, und gegebenenfalls beim Einsetzen eines Deckels eine (9a) der Düsen den Wasserstrahl auch auf die Unterseite des Deckels richtet,

– daß die allseitige Reinigung des Spülgutes durch die Reinigungsdüse (7) über die Leitung (12a) durch die Drehachse zum Reinigungssystem erfolgt, oder durch die weiteren Düsen (4, 4a) in der Spülkammer unterstützt wird,

– daß gereinigte und gegebenenfalls desinfizierte Gefäße als Spülgut entnommen werden können, wenn nach Beendigung des Reinigungsvorganges und Abstellen der Wasserzufuhr, die Klapptür (5) geöffnet wird, und

– daß der Reinigungsvorgang in gleicher Weise wiederholbar ist.

Die Vorrichtung der Erfindung ist weiter dadurch gekennzeichnet,

– daß diese Tür eine Klapptür (5) ist und eine Drehachse (6) an ihrem unteren Rand aufweist, wobei die Klapptür in ihrem inneren Hohlraum ein Rohrleitungssystem (7) mit zwei oder mehreren auf der Innenseite der Tür angeordneten Düsen (9, 9a) und mit einem Verbindungsstück (10) aufweist, und die Klapptür in der Drehachse nach oben zugeklappt, oder motorisch geschlossen wird,

– daß das Verbindungsstück ein auf der Drehachse drehbares Winkelstück ist, das über eine im Spülraum oder nach außen geführte Rohrleitung (12) an das Rohrleitungssystem (7) der Spülkammer (1) angeschlossen ist,

– daß nach dem Einschalten des Reinigungswassers, der Wasserstrahl durch die Düsen vollständig auf die Unterseite des Spülgutes, wie Pflegegefäße (8) gerichtet ist, und gegebenenfalls beim Einsetzen eines Deckels eine (9a) der Düsen den Wasserstrahl auch auf die Unterseite des Deckels richtet,

– daß die allseitige Reinigung des Spülgutes durch die Reinigungsdüse (7) über die Leitung (12a) durch die Drehachse zum Reinigungssystem erfolgt, oder durch die weiteren Düsen (4, 4a) in der Spülkammer unterstützt wird,

– daß gereinigte und gegebenenfalls desinfizierte Gefäße als Spülgut entnommen werden können, wenn nach Beendigung des Reinigungsvorganges und Abstellen der Wasserzufuhr, die Klapptür (5) geöffnet wird, und

– daß der Reinigungsvorgang in gleicher Weise wiederholbar ist.

Die Vorrichtung der Erfindung ist auch dadurch gekennzeichnet, daß das Rohrleitungssystem (7) eine Düse (14) aufweist, die auf einem nach oben und dann nach unten abgebogenen Rohrstück (13) so angeordnet ist, daß eine zu reinigende Flasche (8a), wie eine Urin- oder Sekret-Flasche, bei noch offener Klapptür (5) auf die Düse (14) aufgeschoben wird und dadurch, daß das Hygienegefäß (8) und/oder der Deckel und/oder eine oder zwei Flaschen, wie Urin- oder Sekret-Flaschen, in eine Haltevorrichtung eingeschoben werden, die das Spülgut bei dem Reinigungsvorgang in etwa senkrechter Stellung hält.

Die Vorrichtung der Erfindung ist auch durch ein Verfahren zu seiner Benutzung gekennzeichnet,

– dass bei aufgeklappter Tür (5) der Spülkammer (1) in die Befestigungshalterung ein oder mehrere Pflegegefäße (8, 8a) eingesetzt und diese dabei gleichzeitig über die Düsen (14) am Ende des gekrümmten Rohres (15) geschoben werden, und dadurch zwangsläufig die Düsen in den Flaschenhälsen zentriert werden, und danach die Klapptür (5) im Drehpunkt (6) nach oben zugeklappt wird, und danach durch eine, an sich bekannte Programmsteuerung durch das Rohrleitungssystem (7) Reinigungswasser durch die Düsen (9, 9a, 9b) in die Spülkammer (1) eingedüst und durch die an dieses Rohrleitungssystem (7) angeschlossene Düse (14) gleichzeitig Reinigungswasser bei und nach der Entleerung der Pflegegefäße eingespritzt wird und die allseitige Reinigung des Spülgutes durch die Reinigungsdüse (17) über die Verbindungsleitung (16) zum Reinigungssystem (7), oder durch die weiteren Düsen (4, 4a) in der Spülkammer unterstützt wird, und das Reinigungswasser und vorher der Inhalt der Pflegegefäße durch den konischen Unterteil (2) und über den Geruchverschluß (3) abläuft, wobei das Rohrleitungssystem (7) über das Verbindungsstück (10) beim Zuklappen der Tür (5) in den Verbindungsstutzen (11) eingeführt wird und durch die Rohrleitungen (12) und (13) das Reinigungswasser den Düsen (17) oder (4, 4a) sowie (9, 9a, 9b) zugeführt wird, und nach Beendigung der Entleerung und Reinigung und gegebenenfalls Desinfektion die Klapptür (5) aufgeklappt, die gereinigten Pflegegefäße, wie Urin- und/oder Sekret-Flaschen, entnommen und diese Verfahrensstufen wiederholt werden.

Die Vorrichtung der Erfindung wird durch die Figuren erläutert.

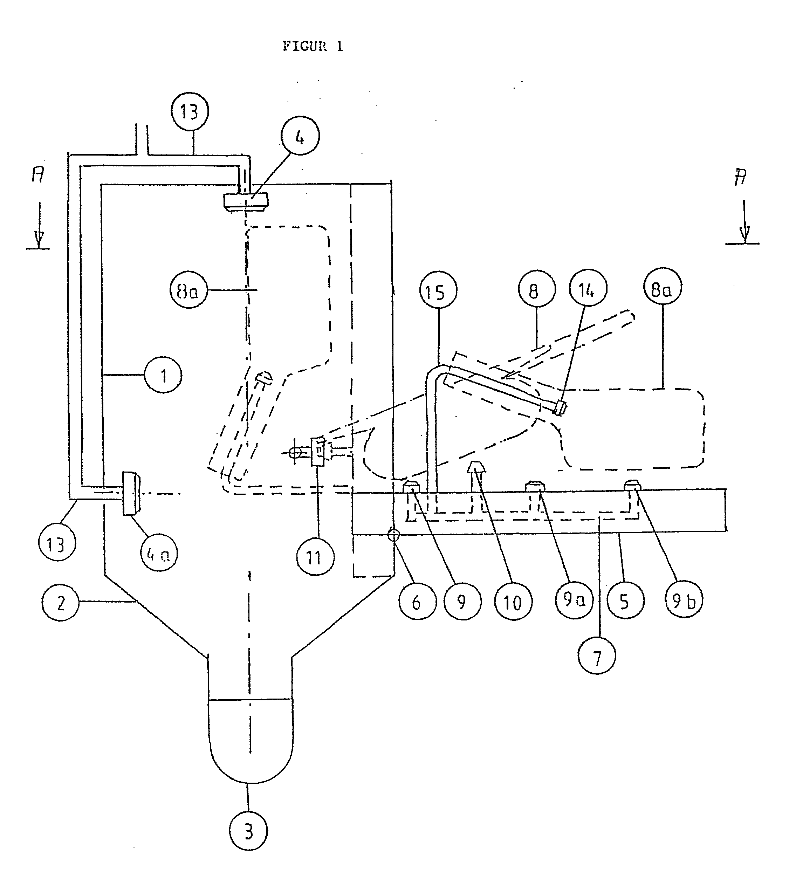

Figur 1 stellt die Seitenansicht mit waagerecht aufgeklappter Tür dar.

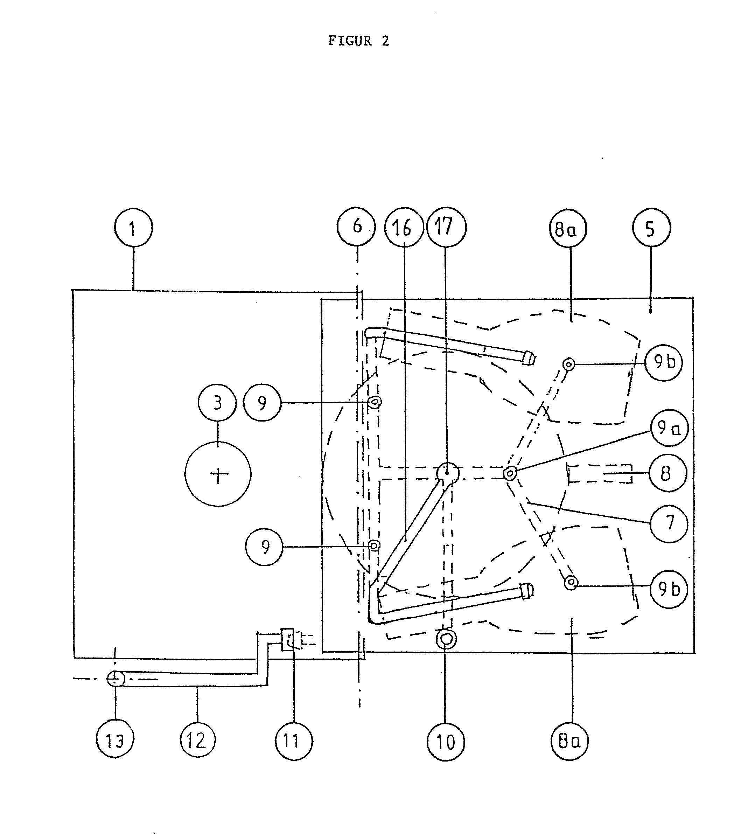

Figur 2 stellt die Aufsicht mit der Schwenkbewegung des Hygienegefäßes dar.

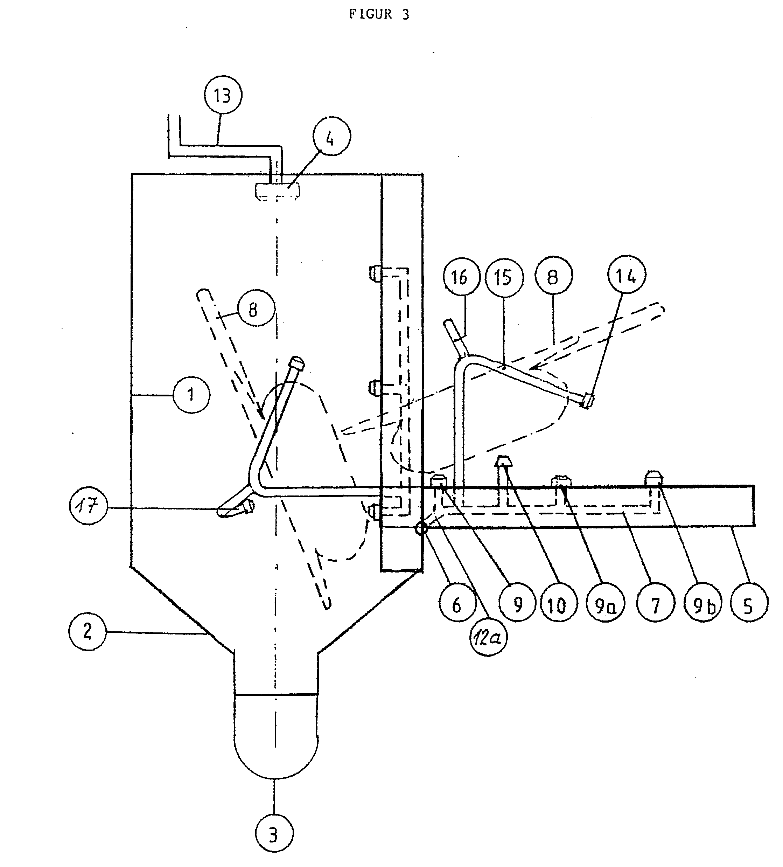

Figur 3 stellt die Seitenansicht mit Schwenkbewegung des Steckbeckens dar.

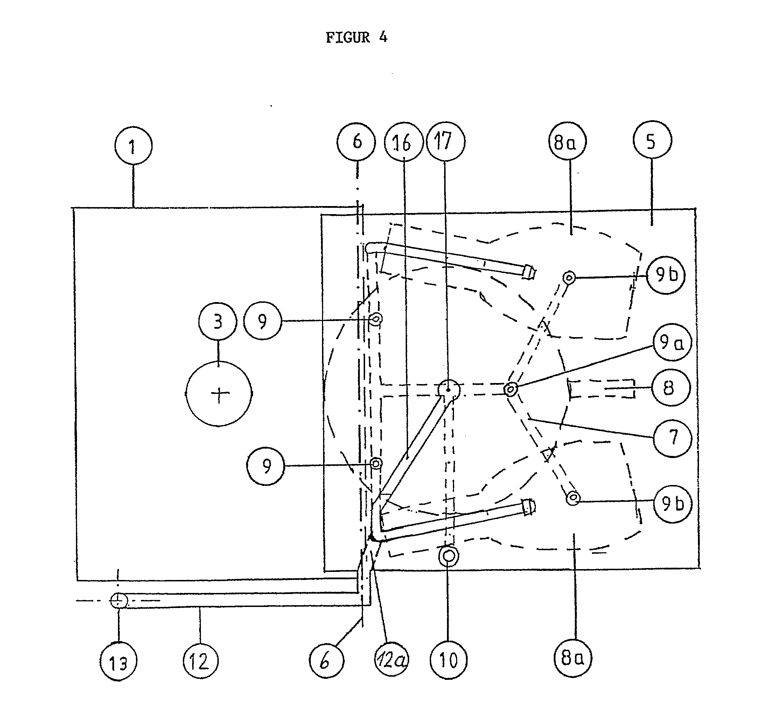

Figur 4 stellt die Aufsicht der Wasserzuführung durch die Drehachse der Tür dar.

In den Figuren bedeuten die Ziffern:

| Ziffer | Teil |
|--------|------|
| 1 | Spülkammer |
| 2 | konischer Unterteil |
| 3 | Geruchabschluß |
| 4, 4a | Reinigungsdüsen |
| 5 | Klapptür |
| 6 | Drehpunkt |
| 7 | Rohrleitungssystem |
| 8, 8a | Pflegegefäße |
| 9, 9a, 9b | Düsen |
| 10 | Verbindungsstück |
| 11 | Verbindungsstutzen |
| 12, 12a | Rohrleitung |
| 13 | Rohrleitung |
| 14 | Düse |
| 15 | gekrümmte Rohrleitung |
| 16 | Verbindungsleitung zum Rohrsystem (7) |
| 17 | Reinigungsdüse |

Soweit Vorrichtungen mit Klappdeckel verwendet werden, ist bei diesen die Düse in der Spülkammer angeordnet. Es ist durch diese konstruktive Ausbildung keine vollständige Reinigung aller Flächen des Spülgutes möglich. Es bleibt die Fläche vom Reinigunswasser unberührt, die dem Klappdeckel zugewandt ist.

Es ist damit auch keine sichere Innenreinigung der eingesetzten Flache möglich.Bei Flachen mit kleinem Flaschenhals bleibt die Düse beim Zuklappen ausserhalb der Flaschenöffnung.Bei grösserer Öffnung wird die Düse unzentriert in diese eingeführt.Die Reinigung der Flasche wird dadurch unsicher und unvollständig.

Diese Nachteile werden durch die Vorrichtung der Erfindung vermieden. Diese reinigt das Spülgut allseitig vollständig und zwar die Aussen- und die Innenflächen in optimal kurzer Zeit.

Die Vorrichtung der Erfindung ist auch bevorzugt durch ein Verfahren zu seiner Benutzung gekennzeichnet.Dieses Verfahren ist dadurch gekennzeichnet,dass bei aufgeklappter Tür (5) der Spülkammer (1) in die Befestigungshalterung ein oder mehrere Pflegegefässe (8,8a) eingesetzt und diese dabei gleichzeitig über die Düsen (14) am Ende des gekrümmten Rohres (15) geschoben werden,und dadurch zwangsläufig die Düse in den Flaschenhälsen zentriert sind, und danach die Klapptür (5) im Drehpunkt (6) nach oben zugeklappt wird, und danach durch eine,an sich bekannte Programmsteuerung durch das Rohrleitungssystem (7) Reinigungswasser durch die Düsen (9,9a,9b) in die Spülkammer (1) eingedüst und durch die an dieses Rohrleitungssystem (7) angeschlossene Düse (14) gleichzeitig Reinigungswasser bei und nach der Entleerung der Pflegegefässe eingespritzt wird und die allseitige Reinigung des Spülgutes durch die Reinigungsdüse (17) über die Verbindungsleitung (16) zum Reinigungssystem (7), oder durch die Leitung (12 a) und durch den Drehpunkt (6) zum Reinigungssystem (7) erfolgt, oder durch die weiteren Düsen (4,4a) in der Spülkammer unterstützt wird, und das Reinigungswasser und vorher der Inhalt der Pflegefässe durch den konischen Unterteil (2) und über den Geruchverschluss (3) abläuft, wobei das Rohrleitungssystem (7) über das Verbindungsstück (10) beim Zuklappen der Tür (5) in den Verbindungsstutzen (11) eingeführt wird und durch die Rohrleitungen (12) und (13) das Reinigungswasser den Düsen (17) oder (4,4a), sowie (9,9a,9b) zugeführt wird,und nach Beendigung der Entleerung und Reinigung und gegebenenfalls Desinfektion die Klapptür (5) aufgeklappt, die gereinigten Pflegegefässe,wie Urin- und/ oder Sekret- Flaschen,entnommen und diese Verfahrensstufen wiederholt werden.

Das Verfahren der Erfindung verwendet anstelle der Pflegegefässe in Form von Urin- oder Sekret-Flaschen Steckbecken , die in eine andere Befestigungshalterung eingesetzt werden.

Das Verfahren der Erfindung verwendet gegebenenfalls ein solches Rohr /15) mit Düse (14), dass zusätzlich zur Düse (4a) in die Innenseite des Steckbeckens sprüht und dadurch sicher etwaige, festere Anbackungen entfernt.

Die Vorrichtung der Erfindung mit dem Verfahren zu seiner Durchführung wird durch das folgende Ausführungsbeispiel erläutert.

Eine Pflegeperson öffnet durch Knopdruck mit dem Ellenbogen motorisch die Klapptür (5) und setzt 2 gefüllte Urinflaschen in die Befestigungshalterung ein,die dabei gleichzeitig über die Düsen (14) am Ende des gekrümten Rohres (15) geschoben und zentriert werden. Danach wird durch weiteren Knopfdruck die Klapptür (5) motorisch geschlossen und durch die motorische Drehung der Klapptür (5)das Verbindungsstück (10) in den Verbindungsstutzen (11) eingedrückt, danach wird durch die Programmsteuerung Reinigungswasser durch die Rohrleitungen (7,13) in die Düsen (4,4a,9,9a,9b) in den Spülraum (1) und in die Urinflaschen durch die Düse (14) eingesprüht,nachdem durch die Drehung der Klapptür (5) bereits Urinflüssigkeit in den konischen Unterteil (2) und durch den Geruchverschluss (3) abgelaufen ist,und das Reinigungswasser dadurch ebenfalls abläuft.

Die Reinigungs- und Spülzeit beträgt 25 sec, und diese kann gegebenenfalls durch Änderung der Zeiteinstellung verlängert werden.

Es wird danach eine Desinfektion mit Dampf mit der zusätzlich eingebauten Vorrichtung und nach dem Verfahren des europäischen Patentes 0 093 846 mittels der Prozesssteuerung durchgeführt. Danach wird durch Knopfdruck die Klapptür (5) motorisch geöffnet, und es werden die entleerten und gereinigten,sowie zusätzlich desinfizierten Urinflachen entnommen.

Der Gesamtvorgang vom Einsetzen bis zur Entnahme der Urinflaschen beträgt ohne die Desinfektion 40 sec und mit der alternativen Durchführung der Desinfektion 240 sec.

Die Vorrichtung der Erfindung mit dem Verfahren zu seiner Durchführung bietet den Vorteil der allseitigen und sicheren, kurzzeitigen Reinigung des eingeführten Spülgutes, wie Flaschen oder Steckbecken, Dieser Vorteil besteht auch darin,dass eine vollständige Innen- und Aussen-Reinigung der eingesetzten Flaschen oder Steckbecken kurzzeitig mit optimaler Wassermenge erfolgt.

Es wird für den Reingiungsvorgang nur etwa 25 Liter Wasser benötigt.

Der technische Vorteil liegt auch darin, dass der konstruktive Aufbau der Vorrichtung der Erfindung keine Drehvorrichtung mit Bewegungsmechanismus und Steuerung benötigt. Der konstruktive Aufbau der Vorrichtung der Erfindung ist einfacher,und dieser erfordert somit geringere Investierungskosten.

Ein weiterer Vorteil besteht darin,dass mittels der Befestigungshalterung mehrere Flaschen,insbesondere zwei Flaschen, gleichzeitig eingesetzt werden können und sich dadurch die Durchsatzkapazität erhöhen lässt.

**Patentansprüche**

1. Vorrichtung zur Entleerung und Reinigung von Hygienegefäßen aus einer Spülkammer (1) mit konischem Unterteil (2), mit Geruchabschluß (3), sowie mit Reinigungsdüsen (4, 4a) in der Spülkammer und mit einer horizontalen oder vertikalen Tür, dadurch gekennzeichnet,
   - daß diese Tür eine Klapptür (5) ist und eine Drehachse (6) an ihrem unteren Rand aufweist, wobei die Klapptür in ihrem inneren Hohlraum ein Rohrleitungssystem (7) mit zwei oder mehreren, auf der Innenseite der Tür angeordneten Düsen (9, 9a) und mit einem Verbindungsstück (10) aufweist, und die Klapptür in der Drehachse nach oben zugeklappt, oder motorisch geschlossen wird,
   - daß das Verbindungsstück in einen Verbindungsstutzen (11) einführbar ist, der über eine im Spülraum oder nach außen geführte Rohrleitung (12) an das Rohrleitungssystem (7) der Spülkammer (1) angeschlossen ist,
   - daß nach dem Einschalten des Reinigungswassers, der Wasserstrahl durch die Düsen vollständig auf die Unterseite des Spülgutes, wie Pflegegefäße (8) gerichtet ist, und gegebenenfalls beim Einsetzen eines Deckels eine (9a) der Düsen den Wasserstrahl auch auf die Unterseite des Deckels richtet,
   - daß die allseitige Reinigung des Spülgutes durch die Reinigungsdüse (7) über die Verbindungsleitung (16) zum Reinigungssystem erfolgt, oder durch die weiteren Düsen (4, 4a) in der Spülkammer unterstützt wird,
   - daß gereinigte und gegebenenfalls desinfizierte Gefäße als Spülgut entnommen werden können, wenn nach Beendigung des Reinigungsvorganges und Abstellen der Wasserzufuhr, die Klapptür (5) geöffnet wird, und
   - daß der Reinigungsvorgang in gleicher Weise wiederholbar ist.

2. Vorrichtung zur Entleerung und Reinigung von Hygienegefäßen aus einer Spülkammer (1) mit konischem Unterteil (2), mit Geruchabschluß (3) sowie mit Reinigungsdüsen (4, 4a) in der Spülkammer und mit einer horizontalen oder vertikalen Tür, dadurch gekennzeichnet,
   - daß diese Tür eine Klapptür (5) ist und eine Drehachse (6) an ihrem unteren Rand aufweist, wobei die Klapptür in ihrem inneren Hohlraum ein Rohrleitungssystem (7) mit zwei oder mehreren, auf der Innenseite der Tür angeordneten Düsen (9, 9a) und mit einem Verbindungsstück (10) aufweist, und die Klapptür in der Drehachse nach oben zugeklappt, oder motorisch geschlossen wird,
   - daß das Verbindungsstück ein flexibler Schlauch ist, der über eine im Spülraum oder nach außen geführte Rohrleitung (12) an das Rohrleitungssystem (7) der Spülkammer (1) angeschlossen ist,
   - daß nach dem Einschalten des Reinigungswassers, der Wasserstrahl durch die Düsen vollständig auf die Unterseite des Spülgutes, wie Pflegegefäße (8) gerichtet ist, und gegebenenfalls beim Einsetzen eines Deckels eine (9a) der Düsen den Wasserstrahl auch auf die Unterseite des Deckels richtet,
   - daß die allseitige Reinigung des Spülgutes durch die Reinigungsdüse (7) über die Leitung (12a) durch die Drehachse zum Reinigungssystem erfolgt, oder durch die weiteren Düsen (4, 4a) in der Spülkammer unterstützt wird,
   - daß gereinigte und gegebenenfalls desinfizierte Gefäße als Spülgut entnommen werden können, wenn nach Beendigung des Reinigungsvorganges und Abstellen der Wasserzufuhr, die Klapptür (5) geöffnet wird, und
   - daß der Reinigungsvorgang in gleicher Weise wiederholbar ist.

3. Vorrichtung zur Entleerung und Reinigung von Hygienegefäßen aus einer Spülkammer (1) mit konischem Unterteil (2), mit Geruchabschluß (3) sowie mit Reinigungsdüsen (4, 4a) in der Spülkammer und mit einer horizontalen oder vertikalen Tür, dadurch gekennzeichnet,
   - daß diese Tür eine Klapptür (5) ist und eine Drehachse (6) an ihrem unteren Rand aufweist, wobei die Klapptür in ihrem inneren Hohlraum ein Rohrleitungssystem (7) mit zwei oder mehreren, auf der Innenseite der Tür angeordneten Düsen (9, 9a) und mit einem Verbindungsstück (10) aufweist, und die Klapptür in der Drehachse nach oben zugeklappt, oder motorisch geschlossen wird,
   - daß das Verbindungsstück ein auf der Drehachse drehbares Winkelstück ist, das über eine im Spülraum oder nach außen geführte Rohrleitung (12) an das Rohrleitungssystem (7) der Spülkammer (1) angeschlossen ist,
   - daß nach dem Einschalten des Reinigungswassers, der Wasserstrahl durch die Düsen vollständig auf die Unterseite des Spülgutes, wie Pflegegefäße (8) gerichtet ist, und gegebenenfalls beim Einsetzen eines Deckels eine (9a) der Düsen den Wasserstrahl auch auf die Unterseite des Deckels richtet,
   - daß die allseitige Reinigung des Spülgutes durch die Reinigungsdüse (7) über die Leitung (12a) durch die Drehachse zum Reinigungssystem erfolgt, oder durch die weiteren Düsen (4, 4a) in der Spülkammer unterstützt wird,

– daß gereinigte und gegebenenfalls desinfizierte Gefäße als Spülgut entnommen werden können, wenn nach Beendigung des Reinigungsvorganges und Abstellen der Wasserzufuhr, die Klapptür (5) geöffnet wird, und

– daß der Reinigungsvorgang in gleicher Weise wiederholbar ist.

4. Vorrichtung nach Anspruch 2, dadurch gekennzeichnet, daß das Rohrleitungssystem (7) eine Düse (14) aufweist, die auf einem nach oben und dann nach unten abgebogenen Rohrstück (15) so angeordnet ist, daß eine zu reinigende Flasche (8a), wie eine Urin- oder Sekret-Flasche, bei noch offener Klapptür (5) auf die Düse (14) aufgeschoben wird.

5. Vorrichtung nach den Ansprüchen 1 und 2, dadurch gekennzeichnet, daß das Hygienegefäß (8) und/oder der Deckel und/oder eine oder zwei Flaschen, wie Urin- oder Sekret-Flaschen, in eine Haltevorrichtung eingeschoben werden, die das Spülgut bei dem Reinigungsvorgang in etwa senkrechter Stellung hält.

6. Verfahren zur Benutzung der Vorrichtung nach den Ansprüchen 1 bis 4, dadurch gekennzeichnet, daß bei aufgeklappter Tür (5) der Spülkammer (1) in die Befestigungshalterung ein oder mehrere Pflegegefäße (8, 8a) eingesetzt und diese dabei gleichzeitig über die Düsen (14) am Ende des gekrümmten Rohres (15) geschoben werden, und dadurch zwangsläufig die Düsen in den Flaschenhälsen zentriert werden, und danach die Klapptür (5) im Drehpunkt (6) nach oben zugeklappt wird, und danach durch eine, an sich bekannte Programmsteuerung durch das Rohrleitungssystem (7) Reinigungswasser durch die Düsen (9, 9a, 9b) in die Spülkammer (1) eingedüst und durch die an dieses Rohrleitungssystem (7) angeschlossene Düse (14) gleichzeitig Reinigungswasser bei und nach der Entleerung der Pflegegefäße eingespritzt wird und die allseitige Reinigung des Spülgutes durch die Reinigungsdüse (17) über die Verbindungsleitung (16) zum Reinigungssystem (7), oder durch die weiteren Düsen (4, 4a) in der Spülkammer unterstützt wird, und das Reinigungswasser und vorher der Inhalt der Pflegegefäße durch den konischen Unterteil (2) und über den Geruchverschluß (3) abläuft, wobei das Rohrleitungssystem (7) über das Verbindungsstück (10) beim Zuklappen der Tür (5) in den Verbindungsstutzen (11) eingeführt wird und durch die Rohrleitungen (12) und (13) das Reinigungswasser den Düsen (17) oder (4, 4a) sowie (9, 9a, 9b) zugeführt wird, und nach Beendigung der Entleerung und Reinigung und gegebenenfalls Desinfektion die Klapptür (5) aufgeklappt, die gereinigten Pflegegefäße, wie Urin- und/oder Sekret-Flaschen, entnommen und diese Verfahrensstufen wiederholt werden.

## Claims

1. Device for the emptying and cleaning of hygiene receptables from a rinsing chamber (1) with a conical lower part (2), with odor end (3) as well as with cleaning nozzles (4, 4a) in the rinsing chamber and with a horizontal or vertical door, characterized in that

– this door is a trap door (5) and shows a swivel axis (6) at the lower edge, whereat the inner cavity of the trap door shows a pipeline system (7) with two or more nozzles (9, 9a), arranged at the inside of the door, and with a connector (10), and the trap door closes upward in the swivel axis or is motor-driven,

– the connector can be installed into a connecting piece (11) which is connected with the pipeline system (7) of the rinsing chamber (1) via pipe (12) inside the rinsing chamber or external,

– after switching on the cleaning water, the water jet is fully directed through the nozzles to the lower part of the rinsing goods, like receptables (8), and – if needed – by inserting a cover, the water jet is also directed to the lower part of the cover by one of the nozzles (9a),

– the allround cleaning of the rinsing goods is carried out by the cleaning nozzle (7) via connecting pipe (16) to the cleaning system or is supported by further nozzles (4, 4a) in the rinsing chamber,

– cleaned and – if needed – disinfected receptables can be removed as rinsing goods, when the trap door (5) is opened after the cleaning process and stopping of the water supply, and

– the cleaning process can be repeated in the same way.

2. Device for the emptying and cleaning of hygiene receptables from a rinsing chamber (1) with conical lower part (2), with odor end (3) as well as with cleaning nozzles (4, 4a) in the rinsing chamber and with a horizontal or vertical door, characterized in that

– this door is a trap door (5) and shows a swivel axis (6) at the lower edge, whereat the inner cavity of the trap door shows a pipeline system (7) with two or more nozzles (9, 9a), arranged at the inside of the door, and with a connector (10), and the trap door closes upward in the swivel axis or is motor-driven,

– the connector is a flexible tube which is connected with the pipeline system (7) of the rinsing chamber via pipe (12) inside the rinsing chamber or external,

– after switching on the cleaning water, the water jet is fully directed through the nozzles to the lower side of the rinsing goods, like receptables (8), and – if needed – by inserting a cover, the water jet is also directed to the lower part of the cover by one of the nozzles (9a),

– the allround cleaning of the rinsing goods is carried out by the cleaning nozzle (7) via pipe (12a) by the swivel axis to the cleaning system or is supported by further nozzles (4, 4a) in the rinsing chamber,

– cleaned and – if needed – disinfected receptables can be removed as rinsing goods, when the trap door (5) is opened after the cleaning process and stopping of the water supply, and

– the cleaning process can be repeated in the same way.

3. Device for the emptying and cleaning of hygiene receptables from a rinsing chamber (1) with a conical lower part (2), with odor end (3) as well as with cleaning nozzles (4, 4a) in the rinsing chamber and with a horizontal or vertical door, characterized in that

— this door is a trap door (5) and shows a swivel axis (6) at the lower edge, whereat the inner cavity of the trap door shows a pipeline system (7) with two or more nozzles (9, 9a), arranged at the inside of the door, and with a connector (10), and the trap door closes upward in the swivel axis or is motor-driven,

— the connector is an angle plate rotating on the swivel axis, which is connected with the pipeline system (7) of the rinsing chamber (1) via pipe (12) inside the rinsing chamber or external,

— after switching on the cleaning water, the water jet is fully directed to the lower part of the rinsing goods, like receptables (8), through the nozzles and, if needed — by inserting a cover, the water jet is also directed to the lower part of the cover by one of the nozzles (9a),

— the allround cleaning of the rinsing goods is carried out by the cleaning nozzle (7) via pipe (12a) by the swivel axis to the cleaning system or is supported by further nozzles (4, 4a) in the rinsing chamber,

— cleaned and — if needed — disinfected receptables can be removed as rinsing goods, when the trap door is opened after the cleaning process and stopping of the water supply, and

— the cleaning process can be repeated in the same way.

4. Device according to claim 2, characterized in that the pipeline system (7) shows a nozzle (14), which is arranged on a socket (15) deflected upward and downward in such a way that a bottle (8a) to be cleaned — like urine or secretion bottle, is pushed on the nozzle (14), while the trap door (5) is still open.

5. Device according to claims 1 and 2, characterized in that the hygiene receptables (8) and/or the cover and/or one or two bottles, like urine or secretion bottles, can be pushed into a stop device which keeps the rinsing goods in about vertical position during the cleaning process.

6. Process for the use of the device according to claims 1 to 4, characterized in that one or more receptables (8, 8a) can be put into the mounting support, and, at the same time, can be pushed over the nozzles (14) at the end of the bent pipe (15) while the door (5) of the rinsing chamber (1) is open, thus necessarily centering the nozzles in the bottlenecks, and thereafter the trap dor (5) is closed upward in the swivel point (6), and thereafter cleaning water is sprayed through nozzles (9, 9a, 9b) into the rinsing chamber through pipeline system (7) by a known program device, and, at the same time, cleaning water is injected through nozzle (14) attached to pipeline system (7) during and after the emptying of the receptables, and the allround cleaning of the rinsing goods is supported through nozzle (17) via connection pipe (16) to the cleaning system (17) or by further nozzles (4, 4a) in the rinsing chamber, and the cleaning water, and before, the contents of the receptables, leave through the conical lower part (2) via odor end (3), whereat the pipeline system (7) is inserted into the connecting piece (11) via connector (10) when shutting the door, and the cleaning water is directed to nozzles (17) or (4, 4a) as well as (9, 9a, 9b) via pipes (12) and (13), and the trap door (5) is opened after the emptying and cleaning and — if needed — disinfection, the cleaned receptables, like urine and/or secretion bottles, are removed, and these process steps are repeated.

**Revendications**

1. Appareil pour la vidange et nettoyage des récipients d'hygiène d'une chambre de lavage (1) avec une partie inférieure conique (2), avec siphon (3) ainsi que avec des buses de nettoyage (4, 4a) dans la chambre de lavage et avec une porte horizontale ou verticale, caractérisé en ce que

— cette porte est une trappe (5) et présente un axe de rotation (6) au bord inférieur, où la trappe présante dans la cavité intérieure un système de tuyaux (7) avec deux ou plusieurs buses (9, 9a), placées au côté intérieur de la porte, et avec une pièce de jonction (10), et la trappe est fermée dans l'axe de rotation en haut ou par commande moteur,

— on peut pousser la pièce de jonction (10) dans une tubulure (11), attachée au système de tuyaux (7) de la chambre de lavage (1) par l'intermédiaire d'un conduit (12), se trouvant dans la chambre de lavage ou extérieur,

— après le démarrage de l'eau de nettoyage, le jet d'eau est dirigé par des buses complètement vers la face inférieure des récipients (8) et, le cas échéant, si un couvercle est installé, une des buses (9a) dirige le jet d'eau aussi vers la face inférieure du couvercle,

— le nettoyage universel des récipients se produit par la buse de nettoyage (7) par l'intermédiaire de la ligne de connexion (16) au système de nettoyage ou est supporté par des buses (4, 4a) dans la chambre de lavage,

— on peut enlever des récipients nettoyés et — le cas échéant — désinfectés, quand la trappe est ouverte après le procédé de nettoyage est après l'arrêt d'amenée d'eau,

— on peut répéter le procédé de même manière.

2. Appareil pour la vidange et nettoyage des récipients d'hygiène d'une chambre de lavage (1) avec une partie inférieure conique (2), avec siphon (3) ainsi que avec des buses de nettoyage (4, 4a) dans la chambre de lavage et avec une porte horizontale ou verticale, caractérisé en ce que

— cette porte est une trappe (5) et présente un axe de rotation (6) au bord inférieur, où la trappe présente dans la cavité intérieure un système de tuyaux (7) avec deux ou plusieurs buses (9, 9a), placées au côté intérieur de la porte, et avec une pièce de jonction (10), et la trappe est fermée dans l'axe de rotation en haut ou par commande moteur,

— la pièce de jonction (10) est un tuyau flexible, attaché au système de tuyaux (7) de la chambre de lavage (1) à l'intermédiaire d'un conduit (12) se trouvant dans la chambre de lavage ou extérieur,

– après le démarrage de l'eau de nettoyage, le jet d'eau est dirigé par des buses complètement vers la face inférieure des récipients (8) et – le cas échéant – si un couvercle est installé, une des buses (9a) dirige le jet d'eau aussi vers la face inférieure du couvercle,

– le nettoyage universel des récipients se produit par la buse de nettoyage (7) par l'intermédiaire du tuyau (12a) au système de nettoyage par l'axe de rotation ou est supporté par des buses (4, 4a) dans la chambre de lavage,

– on peut enlever des récipients nettoyés et – le cas échéant – désinfectés, quand la trappe est ouverte après le procédé de nettoyage est après l'arrêt d'amenée d'eau,

– on peut répéter le procédé de même manière.

3. Appareil pour la vidange et nettoyage des récipients d'hygiène d'une chambre de lavage (1) avec une partie inférieure conique (2), avec siphon (3) ainsi que avec des buses de nettoyage (4, 4a) dans la chambre de lavage, et avec une porte horizontale ou verticale, caractérisé en ce que

– cette porte est une trappe (5) et présente un axe de rotation (6) au bord inférieur, où la trappe présente dans la cavité intérieure un système de tuyaux (7) avec deux ou plusieurs buses (9, 9a), placées au côté intérieur de la porte, et avec une pièce de jonction (10), et la trappe est fermée dans l'axe de rotation en haut ou par commande moteur,

– la pièce de jonction (10) est un coude rotatif sur l'axe de rotation, attaché au système de tuyaux (7) par l'intermédiaire d'un conduit (12) se trouvant dans la chambre de lavage ou extérieur,

– après le démarrage de l'eau de nettoyage le jet d'eau est dirigé par des buses complètement vers la face inférieure des récipients (8) et – le cas échéant – si un couvercle est installé, une des buses (9a) dirige le jet d'eau aussi vers la face inférieure du couvercle,

– le nettoyage universel des récipients se produit par la buse de nettoyage (7) par l'intermédiaire d'un conduit (12a) par l'axe de rotation au système de nettoyage ou est supporté par des buses (4, 4a) dans la chambre de lavage,

– on peut enlever des récipients nettoyés et – le cas échéant – désinfectés, quand la trappe est ouverte après le procédé de nettoyage est après l'arrêt d'amenée d'eau,

– on peut répéter le procédé de même manière.

4. Appareil selon revendication 2, caractérisé en ce que le système de tuyaux (7) présente une buse (14), placée sur une tubulure courbée en haut et en bas de manière qu'on peut pousser un flacon (8a), comme un flacon à urine ou un flacon à secret, au-dessus la buse (14), quand la trappe est encore ouverte.

5. Appareil selon les revendications 1 et 2, caractérisé en ce qu'on peut pousser le récipient (8) et/ou le couvercle et/ou un ou deux flacons, comme flacons à urine ou flacons à secret, dans un support de fixation tenant le récipient pendant le nettoyage environ en position verticale.

6. Procédé pour l'usage de l'appareil selon les revendications 1 à 4, caractérisé en ce qu'on met un ou plusieurs récipients (8, 8a) dans le support de fixation, quand la porte (5) de la chambre de lavage est ouverte, et en même temps, les on pousse par-dessus les buses (14) au bout du tuyau courbé (15), et par ça les buses sont nécessairement mis au centre dans les goulots, et ensuite la trappe (5) est fermée en haut dans le centre de rotation, et ensuite eau de nettoyage est injecté dans la chambre de lavage au moyen des buses (9, 9a, 9b) par réglage de programme connu par l'intermédiaire du système de tuyaux (7) et en même temps par la buse (14), attaché à cet système de tuyaux (7), eau de nettoyage est injectée pendant et après la vidange des récipients, et le nettoyage universel des récipients est supporté par la buse de nettoyage (17) par l'intermédiaire d'une ligne de connexion (16) au système de tuyaux (7) ou par des buses (4, 4a) dans la chambre de lavage, et l'eau de nettoyage, et avant le contenu des récipients, écoulent à travers la partie inférieure conique (2) au moyen le siphon (3), où on pousse le système de tuyaux (7) au-dessus la pièce de jonction (10) dans la tubulure (11) en fermant la porte, et l'eau de nettoyage est dirigé au moyen des conduits (12, 13) aux buses (17) ou (4, 4a) ou (9, 9a, 9b), et la trappe est ouverte après la vidange et nettoyage, et – le cas échéant – après désinfection des récipients nettoyés, comme flacons à urine et/ou flacons à secret, sont enlevés, et ces grades de procédé sont répétés.

FIGUR 1

FIGUR 2

FIGUR 3

EP 0 294 516 B1

FIGUR 4